# EUROPEAN PATENT SPECIFICATION

(11) **EP 4 041 160 B1**
(45) Date of publication and mention of the grant of the patent: **01.05.2024**
(21) Application number: 20799822.0
(22) Date of filing: 06.10.2020
(51) Int. Cl.: A61F 13/00, A61F 15/02

(54) **CUTTING AID FOR WOUND THERAPY SYSTEMS**
SCHNEIDHILFE FÜR WUNDTHERAPIESYSTEME
AIDE À LA COUPE POUR SYSTÈMES DE TRAITEMENT DE PLAIES

(30) Priority: 08.10.2019 US 201962912167 P
(43) Date of publication of application: 17.08.2022
(73) Proprietor: Solventum Intellectual Properties Company, Maplewood, MN 55144 (US)
(72) Inventor: SEDDON, James Killingworth, San Antonio, Texas 78265 (US); PRATT, Benjamin A., San Antonio, Texas 78265 (US); RICE, Justin, San Antonio, Texas 78265 (US)
(74) Representative: Simmons & Simmons
(86) International application number: PCT/IB2020/059376
(87) International publication number: WO 2021/070053

(56) References cited:
- WO-A1-2013/043972
- US-A1- 2011 152 798

## Description

### BACKGROUND

The present disclosure relates generally to wound therapy systems and devices, and more particularly to a cutting aid for negative pressure wound therapy systems, methods, and devices.

Negative pressure wound therapy (NPWT) is a type of wound therapy that involves applying negative pressure (relative to atmospheric pressure) to a wound site to promote wound healing. Some NPWT systems surround the wound in a dressing which is sealed with a drape. The drape establishes a barrier between negative pressure and atmospheric pressure. The negative pressure is created by a pump which provides suction through a tube to a pad which is coupled to the dressing over the wound site.

The application of the dressing for the NPWT systems can be difficult for challenging wound sites and wound types. Once a dressing has been placed over the wound and the drape is applied to provide an occlusive cover, a small hole has to be cut through the top of the drape to allow connectivity with the pad. It would be desirable to provide a device that facilitates an easier and more accurate way to cut the hole through the drape.
WO2013/043972 discloses a negative pressure wound therapy system including a flexible member and a conduit for coupling reduced pressure to the wound, wherein a section of the conduit has a cutting edge for cutting an evacuation port in the flexible membrane.
US2011/152798 discloses a vacuum therapy system with a cutting-to-size device to allow a wound shape to be traced and transferred to a liquid-permeable dressing element.

### SUMMARY

The invention is defined by the claims and comprises a method of cutting a drape for use with a negative pressure wound therapy device. The method includes: placing a cutting aid device on the drape, lifting up the cutting aid device to lift up a portion of the drape, and cutting the lifted portion of the drape. The cutting aid device includes a holding section and a contact section. The contact section includes a top surface connected to the holding section, and a bottom surface with an adhesive.
Insofar as the term invention or embodiment is used in the following, or features are presented as being optional, this should be interpreted in such a way that the only protection sought is that of the invention claimed.
Reference(s) to "embodiment(s)" throughout the description which are not under the scope of the appended claims merely represent possible exemplary executions and are not part of the present invention.

### BRIEF DESCRIPTION OF THE DRAWINGS

Various objects, aspects, features, and advantages of the disclosure will become more apparent and better understood by referring to the detailed description taken in conjunction with the accompanying drawings, in which like reference characters identify corresponding elements throughout. In the drawings, like reference numbers generally indicate identical, functionally similar, and/or structurally similar elements.
FIG. 1 is a diagram of a NPWT system shown according to an illustrative embodiment.
FIG. 2 is a diagram of a cutting aid device shown according to an illustrative embodiment.
FIG. 3 is a diagram of a cutting aid device shown according to an illustrative embodiment.
FIG. 4 is a diagram of a kit shown according to an illustrative embodiment.
FIG. 5 is a flow diagram of a method of cutting a drape for use with a NPWT system using a cutting aid device shown according to an illustrative embodiment.

### DETAILED DESCRIPTION

Cutting a recommended size hole in a drape layer without disrupting the underlying wound dressing and potentially causing a leak while applying a NPWT system is challenging and requires skill and knowledge. The drape adhesive is often firmly attached to the dressing surface. If the hole is cut smaller than the recommended size, hanging fragments of the drape may be left after connection of the pad over the hole, which can lead to a blockage of the tube of the NPWT system. If the hole is cut larger than the recommended size, the pad cannot connect and seal to the drape fully without a leak of air.

The present disclosure provides systems and methods of a cutting aid (e.g., tool, device, etc.) for providing an easier, faster, and more accurate way to cut a recommended size hole without causing leakage, blockage, or damage to the dressing layer.

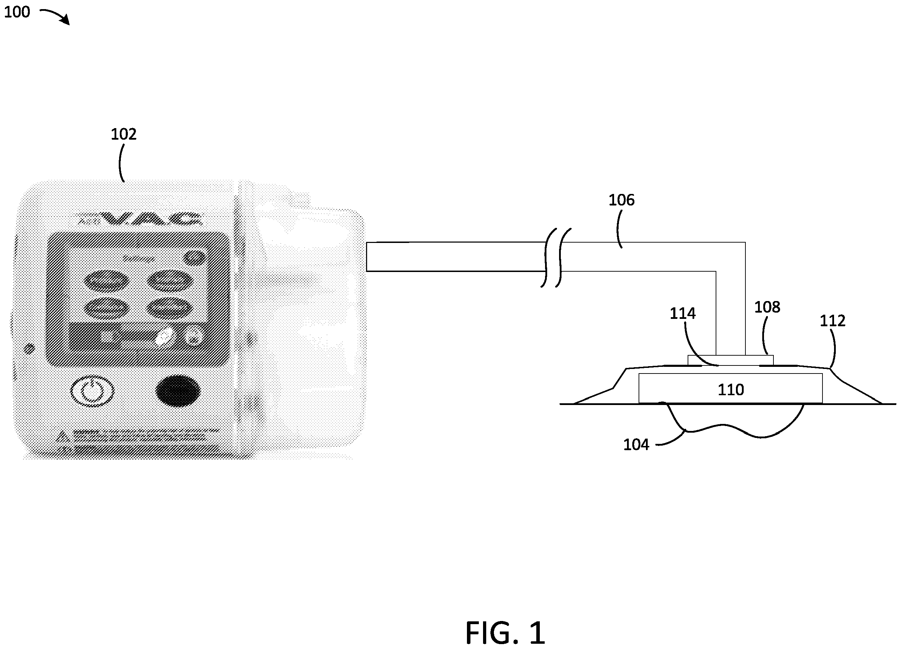

Referring now to FIG. 1, a NPWT system 100 is shown, according to an illustrative embodiment. NPWT system 100 is shown to include a therapy device 102 fluidly connected to a wound site 104 via a tube 106, a dressing layer 110, and an interface pad (such as a V.A.C. VERAT.R.A.C. TM PAD) 108 that fluidly connects the tube 106 to the dressing layer 110. The dressing layer 110 covers a wound site 104. A drape 112 covers the dressing layer 110. The drape 112 typically adheres to the dressing layer 110 and the patient's skin. The drape 112 facilitates a pressure differential between the wound site 104 and a surrounding atmosphere. The drape 112 may be referred to as a drape layer.

The therapy device 102 can be configured to provide negative pressure wound therapy by reducing the pressure at the wound site 104. The therapy device 102 can draw a vacuum at the wound site 104 (relative to atmospheric pressure) by removing wound exudate, air, and other fluids from the wound site 104 through the pad 108 and the tube 106. Wound exudate may include fluid that filters from a patient's circulatory system into lesions or areas of inflammation. For example, wound exudate may include water and dissolved solutes such as blood, plasma proteins, white blood cells, platelets, and red blood cells. Other fluids removed from wound site 104 may include instillation fluid previously delivered to the wound site 104. Instillation fluid can include, for example, a cleansing fluid, a prescribed fluid, a medicated fluid, an antibiotic fluid, or any other type of fluid which can be delivered to wound site 104 during wound treatment. The fluids removed from the wound site 104 pass through the pad 108 and then through the tube 106 and may be collected in a canister that is configured to collect wound exudate and other fluids removed from the wound site 104. In addition to providing removing exudate, air, and other fluids from the wound site 104, the tube 106 may include separate lumens for use by the therapy device 102 to measure pressure within the wound site 104.

In some embodiments, when the NPWT system 100 is set up, the drape layer 112 is adhered to the top of the dressing layer 110, then the pad 108 is adhered to the drape layer 112 over a hole 114. Due to inherent difficulties in cutting a more accurately sized and shaped hole (e.g., the hole 114) in a thin drape layer (e.g., the drape 112) that is adhered to the underlying dressing layer (e.g., the dressing layer 110), various conventional ways often lead to the creation of a hole that is either too large or too small, potentially leading to blockage (e.g., a portion of the drape 112 is sucked into the opening in the pad 108, etc.) or leaking (e.g., a gap exists between the pad 108 and the drape 112, etc.). As a result, the NPWT system 100 cannot operate as desired when the hole is too large, too small, or inaccurately shaped.

Even after the drape 112 has been cut in the conventional ways, it is difficult to remove the cut portion because the drape 112 adheres to the dressing 110. Typically, a forceps is used to grip the cut portion and attempts are made to separate the cut portion from the dressing 110. This may be particularly difficult to achieve if the drape is dense or slick. In addition to being time consuming, removing the cut portion can cause further disturbances to the dressing and wound site.

An advantageous cutting aid device as described in more details below is provided in the present disclosure for setting up the NPWT system 100 in a more accurate, faster, easier, and less expensive way.

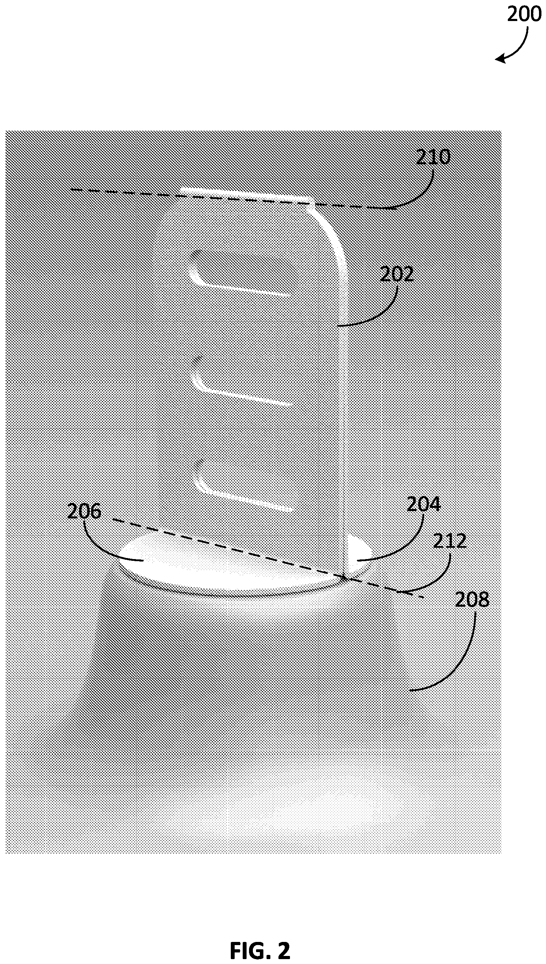

Referring to FIG. 2, a diagram of a cutting aid device 200 is shown according to an illustrative embodiment. The cutting aid device 200 is designed to assist cutting during application of a NPWT system (e.g., the NPWT system 100 of FIG. 1). The cutting aid device 200 includes a holding section 202, a first contact section 204, and a second contact section 206. The cutting aid device 200 may have a die cut two dimensional profile for manufacturing and packaging purposes according to some embodiments. The cutting aid device 200 may be made of any suitable sheet material, such as paper, card stock, polyurethane (PU) film, etc. The sheet material may be adhesive on one side and not adhesive on the other side according to some embodiments. The cutting aid device 200 may be a laminate material which includes a double sided adhesive, such as a double-sided tape (e.g., Lohmann VP20606 tape). The cutting aid device 200 can be customized with any suitable shape, size, and colors. For example, for certain NPWT systems for treating certain wound area, a customized cutting aid device 200 can be provided. The cutting aid device 200 may be embossed with fold lines (e.g., fold lines 210 and 212) to assist with manufacture, packaging, and use. A caregiver (all places) can use the fold lines to fold cutting aid device 200 to form the holding section 202 and the first and second contact sections 204 and 206 before using the cutting aid device 200.

The holding section 202 is used for the caregiver to hold the cutting aid device 200 while cutting a hole on the drape 208. The holding section 202 may be a perpendicular tab according to some embodiments. The holding section 202 may has an angle to the surface of the drape 208. The holding section 202 may include any suitable designs (e.g., embossed patterns, carved patterns) for increasing fraction between the holding section 202 and the caregiver so that the caregiver can more easily grasp the holding section 202 without slipping. The holding section 202 may be made with any suitable shape, size, and colors.

In some embodiments, the holding section 202 is formed by folding the cutting aid device 200 down 90 degrees along the fold line 210. In some embodiments, the cutting aid device 200 is adhesive at one side, so that when it is folded down the two folded adhesive sides along the fold line 210 are attached to each other and form the holding section 202. In some embodiments, the holding section 202 may be may be manufactured with a desired form and does not need to be folded before use.

The first contact section 204 and the second contact section 206 are at the end of the cutting aide device 200. The first contact section 204 and the second contact section 206 are designed to contact with a top surface of the drape 208. The first and the second contact sections 204 and 206 can be formed by folding a first and a second end sections of the cutting aid device 200 up 90 degrees along the fold line 212 after forming the holding section 202. In some embodiments, the first and second contact sections 204 and 206 include an adhesive at the surface that contact with the drape 208. The first and second contact sections 204 and 206 provide adhesive peel force on the contacted drape 208 at a predetermined desired level. According to one embodiment, the adhesive on contact sections 204 and 206 may be an adhesive such as Lohmann DuploCOLL^{®} VP 20606 double sided adhesive tape with a polypropylene film carrier and pure acrylic pressure sensitive adhesive, and a peel strength of approximately 45 N / 25 mm. According to other embodiments, any adhesive with a high bonding strength to low surface energy polymers may be suitable. The adhesive peel force provided by the first and the second sections 204 and 206 on the contacted drape 208 is greater than the peel force of the drape 208 adhesive from the wound filler with the tensile force of the drape 208 material at a pre-determined extension. According to one embodiment, the drape material is a polyurethane film and the adhesive material on the drape that interfaces with the wound filler is a medical grade PSA 1-807 adhesive. The wound filler may be any suitable absorbent materials or a manifolding material, such as a reticulated foam or an open-cell foam, such as those found in a GRANUFOAM^{™} dressing or a V.A.C. VERAFLO^{™} dressing, both available from KCI of San Antonio, Texas. In some embodiments, the first and the second contact sections 204 and 206 form a circular pad to adhesively contact with the top surface of the drape 208. For example, the first and the second contact sections 204 and 206 are folded to form a circular pad having a diameter of approximately 22mm. However, any diameter may be used to suit a particular application.

In some embodiments, the first contact section 204 has same size and shape as the second contact section 206. In other embodiments, the first contact section 204 may have a different size and/or shape compared to the second contact section 206. In some embodiments, the first and the second contact sections 204 and 206 are made of the same material as the holding section 202. In other embodiments, the first and the second contact sections 204 and 206 are made of a different material as the holding section 202. For example, the first and the second contact sections 204 and 206 include a single part release liner to protect the adhesive pad until use. According to other examples, a two-part release liner may be used and is well-suited for use with embodiments of the cutting aid device formed from a flat sheet material, such as the embodiment shown in Figure 2. The two-part release liner would be provided as a kiss-cut release liner (e.g. as present on a roll stock of adhesive) with separately removable portions for (a) the holding section 202, and the contact sections 204 and 206.

In some embodiments, the cutting aid device 200 may be disposable for one-time use. In some other embodiments, the cutting aid device 200 may be reusable. For example, the adhesive side of the first and the second contact sections 204 and 206 may be cleaned for reuse after cutting the hole according to some embodiments. The adhesive side of the first and the second contact sections 204 and 206 may be replaced after use (e.g., replaced by removing and replacing a new double-sided tape).

Once the drape 208 and the dressing 110 have been applied to the wound area, the caregiver may remove the release liner from the first and the second contact sections 204 and 206 of the cutting aid 200 and place the adhesive contact sections 204 and 206 on the drape 208 layer in the location where the caregiver wants to place an interface pad (e.g., a vacuum connection pad) of the NPWT system. The caregiver then may pull (e.g., lift etc.) the cutting aid 200 upward and away from the wound area which subsequently lifts the drape 208 above the dressing layer. The caregiver then may cut the drape 208 just below the cutting aid (e.g., with scissors, etc.) to form a uniform hole with an accurate size that is suitable to use with the interface pad of the NPWT system.

The cutting aid device 200 provides adhesive force over a large area of the drape 208 to distribute the force required to separate the drape 208 from the underlying dressing layer. The large adhesive contact area prevents the drape from tearing. The cutting aid device 200 allows a caregiver to pull away a portion of the drape 208 from the wound area gradually using the adhesive contact force.

Once a desired hole is cut on the drape 208 using the cutting aid device 200, the cut material is adhered to the cutting aid device 200 for removal and disposal. This design eliminates particulates being left behind on the dressing layer. The contact sections 204 and 206 adhered with the cut material can be folded down along the fold line 212 and adhere to each other so that the folded contact section 204 and 206 can be planer with the holding section 202. This design helps to manage the waste material and indicate the cutting aid device 200 has been used.

In some embodiments, the cutting aid device 200 includes a measuring tool (e.g., embossed ruler on the cutting aid device) for measuring a size of a cut hole on the drape 208.

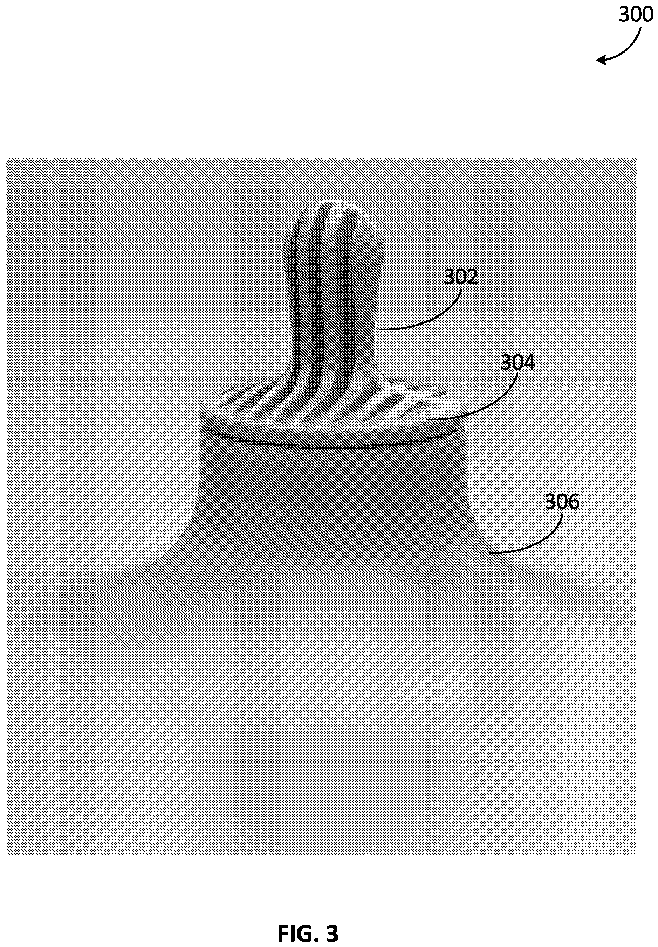

Referring to FIG. 3, a diagram of a cutting aid device 300 is shown according to another illustrative embodiment. The cutting aid device 300 is designed to assist cutting of a drape 306 during application of a NPWT system (e.g., the NPWT system 100 of FIG. 1). The cutting aid device 300 can be used in a similar way as the cutting aid device 200 as described above during application of the NPWT system.

The cutting aid device 300 may be manufactured using 3-D moulding or 3-D printing technologies according to some embodiments. The cutting aid device 300 includes a holding section 302 and a contact section 304. The holding section 302 is designed for the caregiver to grasp the cutting aid device 300. The holding section 302 is arranged vertically on top of the contact section 304. The holding section 302 may be made of any suitable material and may have any desired shapes. The holding section 302 may include embossed patterns to allow the caregiver to easily grip and hold the device.

The contact section 304 is connected to the holding section 302 on the top surface. The contact section 304 includes an adhesive at a bottom surface. The contact section 304 is designed to contact with a top surface of the drape 306. The adhesive bottom surface of the contact section 304 contacts with the drape 306. According to one embodiment, the adhesive on contact section 304 may be an adhesive such as Lohmann DuploCOLL^{®} VP 20606 double sided adhesive tape with a polypropylene film carrier and pure acrylic pressure sensitive adhesive, and a peel strength of approximately 45 N / 25 mm. According to other embodiments, any adhesive with a high bonding strength to low surface energy polymers may be suitable. The adhesive peel force provided by the contact section 304 on the contacted drape 306 is greater than the peel force of the drape 306 adhesive from the wound filler with the tensile force of the drape 306 material at a pre-determined extension. According to one embodiment, the drape material is a polyurethane film and the adhesive material on the drape that interfaces with the wound filler is a medical grade PSA I-807 adhesive. The wound filler may be any suitable absorbent material or a manifolding material, such as a reticulated foam or an open-cell foam, such as those found in a GRANUFOAM^{™} dressing or a V.A.C. VERAFLO^{™} dressing, both available from KCI of San Antonio, Texas. The contact section 304 provides adhesive peel force on the contacted drape 306 at a predetermined desired level. The adhesive peel force provided by the contact section 304 on the contacted drape 306 is greater than the peel force of the drape 306 adhesive from the wound filler with the tensile force of the drape 306 material at a pre-determined extension. In some embodiments, the bottom surface of the contact section 304 has a circular shape. For example, the contact section 304 is circular pad with a diameter approximately 22mm. In other embodiments, the bottom surface of the contact section 304 may have any desired shape and/or size.

In some embodiments, the contact section 304 is made of the same material as the holding section 302. In other embodiments, the contact section 304 is made of different material as the holding section 302. For example, the contact section 304 include a single part release liner to protect the adhesive pad (i.e., the bottom surface) until use. According to other examples, a two-part release liner may be used, which may include two overlapping segments presenting a tab or tail that is free from the adhesive and may be readily grasped and removed from the adhesive by a user.

In some embodiments, the cutting aid device 300 may be disposable for one-time use. In other embodiments, the cutting aid device 300 may be reusable. For example, the adhesive side (i.e., the bottom surface) of the contact section 304 may be cleaned for reuse after cutting the hole according to some embodiments. The adhesive side of the first and the contact section 304 may be replaced after use (e.g., replaced by removing and replacing a new double-sided tape).

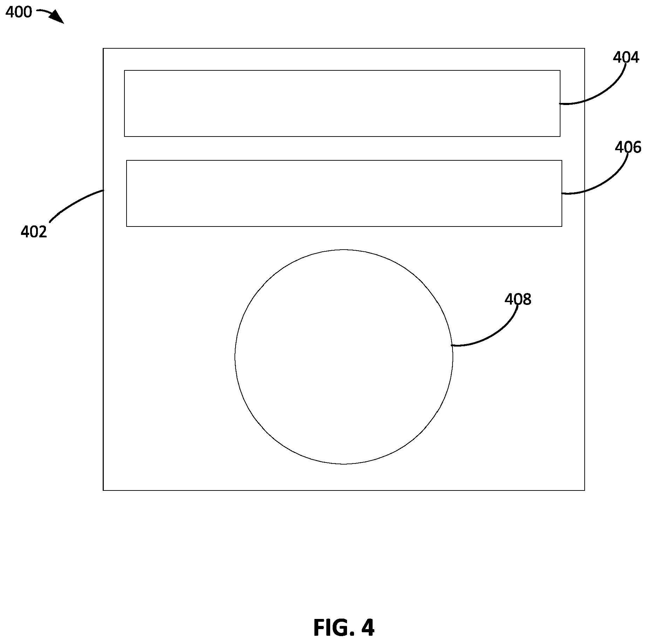

Referring now to FIG. 4, a kit 400 is shown schematically according to an illustrative embodiment. The kit 400 is intended for use with a NPWT device, such as the therapy device 102. The kit 400 includes a package 402. The package 402 defines a sterilized interior environment. The package 402 may be a bag, a pouch, a case, and other similar structures. The kit 400 also includes one or more dressing layers 404. The dressing layer 404 is disposed within package 402. The dressing layer 404 may be any suitable dressing for wound treatment, such as the wound dressing 110 of FIG. 1. The kit 400 also includes a drape layer 406. The drape layer 406 is disposed within the package 402. The drape layer 406 may be any suitable drape used for wound treatment, such as the drape 112 of FIG. 1. The kit 400 also includes a cutting aid device 408. The cutting aid device 408 is disposed within package 402. The cutting aid device 408 may be the cutting aid devices 200 and/or 300.

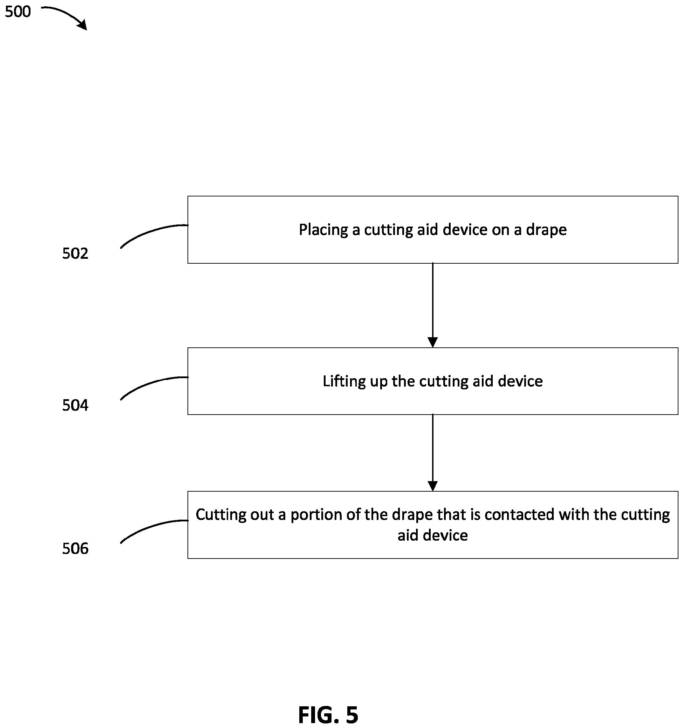

Referring now to FIG. 5, a flow diagram of a method 500 of cutting a drape for use with a NPWT system using a cutting aid device is shown according to an illustrative embodiment. The method 500 may be implemented with a NPWT system, such as the therapy device 102.

At step 502, a cutting aid device is placed on a drape which is disposed on a dressing. The cutting aid device has an adhesive surface that contacts the drape. The adhesive surface bonds to the drape. The adhesive force of the bond applied by the cutting aid device on the drape is greater than the adhesive force of the bond between the drape and the dressing.

At step 504, the cutting aid device is lifted up such that a portion of the drape is lifted up and is separated from the dressing. The greater adhesive force applied by the cutting aid device on the drape enables a portion of the drape to separate from the dressing so that the portion of the drape can be lifted and easily cut.

At step 506, the lifted portion of the drape that contacts with the cutting aid device is cut to form a substantially uniform hole with an accurate size that is suitable to use with the NPWT system. The cut material remains adhered to the cutting aid device. This design helps eliminate particulates from the drape being left behind at the dressing.

According to any of the illustrated embodiments, the cutting aid for an NPWT system provides a number of advantages including, among others, simplified hole cutting process, more quickly separating the drape layer from the dressing layer than conventional methods, ensuring the forming of consistent holes (e.g. 25mm holes), providing a caregiver with an easy hand-hold for use, preventing the drape from prematurely tearing, eliminating the need for a caregiver to cut into the dressing layer, thus eliminating the risk of damaging the dressing layer and reducing manifolding capabilities, eliminating cutting into the dressing layer and leaving particulates in the wound area, managing the drape cut portion ready for disposal. The cutting aid is also low cost, high efficiency, has minimal environmental impact, is disposable and able to be adapted to form any sized holes.

As utilized herein, the terms "approximately," "about," "substantially," and similar terms are intended to have a broad meaning in harmony with the common and accepted usage by those of ordinary skill in the art to which the subject matter of this disclosure pertains. It should be understood by those of skill in the art who review this disclosure that these terms are intended to allow a description of certain features described and claimed without restricting the scope of these features to the precise numerical ranges provided. Accordingly, these terms should be interpreted as indicating that insubstantial or inconsequential modifications or alterations of the subject matter described and claimed are considered to be within the scope of the invention as recited in the appended claims.

It should be noted that the terms "illustrative", "exemplary", and "example" as used herein to describe various embodiments is intended to indicate that such embodiments are possible examples, representations, and/or illustrations of possible embodiments (and such term is not intended to connote that such embodiments are necessarily extraordinary or superlative examples).

The terms "coupled," "connected," and the like, as used herein, mean the joining of two members directly or indirectly to one another. Such joining may be stationary (e.g., permanent, etc.) or moveable (e.g., removable, releasable, etc.). Such joining may be achieved with the two members or the two members and any additional intermediate members being integrally formed as a single unitary body with one another or with the two members or the two members and any additional intermediate members being attached to one another.

References herein to the positions of elements (e.g., "top," "bottom," "above," "below," "between," etc.) are merely used to describe the orientation of various elements in the figures. It should be noted that the orientation of various elements may differ according to other exemplary embodiments, and that such variations are intended to be encompassed by the present disclosure.

The construction and arrangement of the systems and methods as shown in the various exemplary embodiments are illustrative only. Although only a few embodiments have been described in detail in this disclosure, many modifications are possible (e.g., variations in sizes, dimensions, structures, shapes and proportions of the various elements, values of parameters, mounting arrangements, use of materials, colors, orientations, etc.). For example, the position of elements may be reversed or otherwise varied and the nature or number of discrete elements or positions may be altered or varied. Accordingly, all such modifications are intended to be included within the scope of the present disclosure. The order or sequence of any process or method steps may be varied or re-sequenced according to alternative embodiments. Other substitutions, modifications, changes, and omissions may be made in the design, operating conditions and arrangement of the exemplary embodiments without departing from the scope of the present disclosure.

## Claims

1. A method of cutting a drape for use with a negative pressure wound therapy device, the method comprising:
placing a cutting aid device on the drape, wherein the cutting aid device comprises:
a holding section; and
a contact section comprising a top surface connected to the holding section, and an adhesive bottom surface;
lifting up the cutting aid device to lift up a portion of the drape; and
cutting the lifted portion of the drape.

2. The method of claim 1, further comprising applying adhesive force on a top surface of the drape by contacting the bottom surface of the contact section to the top surface of the drape.

3. The method of claim 1, further comprising removing the cut portion of the drape by adhering the portion of the drape to the bottom of the contact section.

## Patentansprüche

1. Ein Verfahren zum Schneiden eines Abdecktuchs zur Verwendung mit einer Unterdruck-Wundtherapievorrichtung, das Verfahren aufweisend:
Platzieren einer Schneidhilfsvorrichtung auf dem Abdecktuch, wobei die Schneidhilfsvorrichtung umfasst:
einen Halteabschnitt; und
einen Kontaktbereich, der eine obere Oberfläche, die mit dem Halteabschnitt verbunden ist, und eine klebende untere Oberfläche aufweist;
Anheben der Schneidhilfsvorrichtung, um einen Abschnitt des Abdecktuchs anzuheben; und
Schneiden des angehobenen Abschnitts des Abdecktuchs.

2. Das Verfahren nach Anspruch 1, ferner aufweisend das Aufbringen einer Klebekraft auf eine obere Oberfläche des Abdecktuchs durch Inkontaktbringen der unteren Oberfläche des Kontaktbereichs mit der oberen Oberfläche des Abdecktuchs.

3. Verfahren nach Anspruch 1, ferner aufweisend das Entfernen des geschnittenen Abschnitts des Abdecktuchs durch Verkleben des Abschnitts des Abdecktuchs mit dem Boden des Kontaktbereichs.

## Revendications

1. Procédé de découpe d'un drapé destiné à être utilisé avec un dispositif de traitement de plaie par pression négative, le procédé comprenant :
le placement d'un dispositif d'aide à la découpe sur le drapé, dans lequel le dispositif d'aide à la découpe comprend :
une section de retenue ; et
une section de contact comprenant une surface supérieure reliée à la section de retenue, et une surface inférieure adhésive ;
le soulèvement du dispositif d'aide à la découpe pour soulever une partie du drapé ; et
la découpe de la partie soulevée du drapé.

2. Procédé selon la revendication 1, comprenant en outre l'application d'une force adhésive sur une surface supérieure du drapé en mettant en contact la surface inférieure de la section de contact avec la surface supérieure du drapé.

3. Procédé selon la revendication 1, comprenant en outre le retrait de la partie découpée du drapé en faisant adhérer la partie du drapé à la partie inférieure de la section de contact.
